# EUROPEAN PATENT APPLICATION

(11) **EP 2 002 811 A1**
(43) Date of publication of application: **17.12.2008**
(21) Application number: 07110066.3
(22) Date of filing: 12.06.2007
(51) Int. Cl.: A61F 5/445, A61F 5/448

(54) **Coupling device and drainage assembly for collecting bodily waste excreted from a body drainage exit, and method of using the coupling and drainage assembly**

(71) Applicant: BioTap A/S, 5700 Svendborg (DK)
(72) Inventor: Rosengren, Oscar, 427 41, Billedal (SE)
(74) Representative: Holme Patent A/S

(57) **Abstract**

A coupling device (1) serves for providing an adhesive body faceplate (32) with means for connecting the body faceplate (32) to a body waste receptacle (18), the body waste plate (32) has an aperture (24) adapted to register with a body drainage exit (30). The coupling device (1) comprises a frame (2) having a central aperture (16) adapted to register with the body drainage exit (30), a magnetic ring (3) mounted in the central aperture (16) of the frame (2) at the internal circumference of the central aperture (16), and that any of the frame (2) or the body faceplate (32) are provided with means (7) for detachable attachment of the coupling device (1) to the body faceplate (32). The coupling device is used for a drainage assembly, such as an ostomy pouch having magnetic attractable means, to provide for magnetic coupling of the ostomy pouch to the abdominal wall using an adhesive body faceplate equipped with the detachable coupling device.

## Description

The present invention relates to a coupling device for providing an adhesive body faceplate with means for connecting the body faceplate to a body waste receptacle, the body waste plate has an aperture adapted to register with a body drainage exit.

Patients with stomas may use either a one-part appliance or a two-part appliance for collecting body waste. In both cases an adhesive body faceplate or wafer is part of the complete appliance. A suitable aperture for the stoma is made in the adhesive body faceplate, and the body faceplate with or without integrated pouch is adhered to the peristomal skin surface to surround the stoma. This adhesion of the body faceplate to the body provides sealing between skin surface and body faceplate so that body waste, such as faeces or urine, does not escape unintentionally but is collected in the pouch.

One disadvantage with one-part appliances is that the entire one-part appliance must be substituted if access to the stoma is required. Another disadvantage is that the pouch wall hides the aperture in the body faceplate, which makes it difficult to register said aperture with the stoma. As a result inexperienced users often misplace the one-part appliance and must start anew with a new one-part appliance until placement is satisfactory and reliable.

The two-part appliance provides an alternative to the one-part appliance. In this case the pouch is designed with an adhesive sheet extending radially from a stoma receiving opening of the pouch, and the body faceplate is a separate part. In the first application step the body faceplate is adhered to the peristomal area and in a subsequent second application step the adhesive sheet of the pouch is adhered on top of the adhesive body faceplate so that the stoma receiving opening of the pouch receives the stoma. The stoma is now located inside the assembled two-part appliance, which now functions in the same manner as the one-part appliance. The body faceplate and the pouch are now inseparable and suffer from the same disadvantages as the one-part appliance. If attempts are made to separate the two parts this results in at least some detachment of the body faceplate from the peristomal skin surface. Thus, impairment of the adhesion and sealing between the skin surface and body faceplate is unavoidable.

International patent application WO2005/048892 describes a coupling between an adhesive body faceplate and an ostomy pouch, i.e. a two-part appliance. The two parts are detachable coupled to each other by means of an interengaging key and slot arrangement. The key and slot are provided as integrated plastic rings on the central part of the body faceplate and around the opening of the pouch, respectively. However, since humidity and body waste has a tendency to accumulate in cavities it is practically impossible to provide for repeated, tight-sealing coupling actions between the two plastic rings. Moreover, the user cannot choose where to make the stoma access opening in the body faceplate since the opening is premade and encircled by the permanently situated, integrated slot. This is of great disadvantage to many ostomy pouch users who prefer to mount the body faceplate eccentric to the stoma in order to provide the best seal at a peristomal area, which has turned out to be more prone to detachment and leakage than another peristomal area. This situation is often due to certain anatomic conditions, such as hernias or cicatricial tissue, which make it very difficult to obtain a good attachment of the body faceplate to a specific part of the skin.

European patent application EP 0 142 259 A1 discloses a body faceplate with a protruding tubular part for surrounding the stoma, and an adhering flange part. An ostomy pouch facing side of the flange part is provided with a permanently embedded integrated magnetic means by means of which the body faceplate can be detachable coupled to the ostomy pouch. The magnetic means consists of a flexible layer impregnated with or otherwise including a magnetic or magnetizable material. This body faceplate according to EP 0 142 259 is expensive to manufacture and does not allow for an individual choice of location of the stoma receiving aperture in the body faceplate. A further disadvantage is that since the magnetic means is integrated in the flange, i.e. exterior to the tubular part, there is a pronounced risk that body waste seeps into the space between the tubular part and the pouch. Finally, the magnetic force of a thin magnetic sheet may prove too small to keep the pouch firmly attached if the magnetic coupling is affected by unintentional mechanical forces, e.g. during the users movements.

The present invention provides improvements of the prior art.

It is a main aspect of the present invention to provide a coupling device of the kind mentioned in the opening paragraph that provides a high degree of freedom of individual design of an adhesive body faceplate.

It is a second aspect of the present invention to provide a coupling device of the kind mentioned in the opening paragraph that can be reused.

It is a third aspect of the present invention to provide a coupling device of the kind mentioned in the opening paragraph which allows for audible coupling together with a body waste receptacle.

It is a fourth aspect of the present invention to provide an alternative drainage assembly for collecting bodily waste excreted from a body drainage exit, which drainage assembly can be more easily coupled and uncoupled than hitherto known.

It is a fifth aspect of the present invention to provide a drainage assembly of the kind mentioned in the opening paragraph which provide a strong, leakproofed coupling between body faceplate and body waste receptacle, which coupling is not sensitive to unintentionally inflicted mechanical forces.

The novel and unique features according to the present invention whereby this is achieved is the fact that the coupling device comprises a frame having a central aperture adapted to register with the body drainage exit, a magnetic ring mounted in the aperture of the frame at the internal circumference of the central aperture, and any of the frame or the body faceplate are provided with means for detachable attachment of the coupling device to the body faceplate.

As used in the present patent application the term "magnetic ring" is to be understood as any closed curve or loop comprising a material, which already is magnetic or can be magnetized, e.g. a solid, ferromagnetic, circular ring. However any closed curve, including polygons, are to be understood as rings.

Magnetic materials are classified in terms of their magnetic properties and their uses. If a material is easily magnetised and demagnetised then it is referred to as a soft magnetic material, whereas if it is difficult to demagnetise then it is referred to as a hard (or permanent) magnetic material. Any of the above magnetic materials are useable for the magnetic ring of the coupling device according to the present invention.

Preferably the magnetic ring is made of a solid, hard ferromagnetic metal or metal alloy, such as Neodyme (NdFeB) or Semarium-Cobolt (SmCo).

When the magnetic ring is mounted in the aperture of the frame at the internal circumference of the central aperture it is ensured that all body waste is passed on to the body waste receptacle in a leakproof manner. The body waste is not able to take any other route than the route directly into the body waste receptacle, and body waste cannot get access to the external circumference of the coupling device. Furthermore, by making the coupling detachable, the expensive magnetic ring can be reused as many times as the means for detachable attachment of the coupling device to the body faceplate provides for without impairing the attachment force and seal.

If the means for detachable attachment of the coupling device to the body faceplate is an adhesive the coupling device is very easy and simple to adhere to the body faceplate at any desired suitable place on the body faceplate. Suitable adhesives, which allows for numerous attachments and detachments of the frame without affecting and impairing the adhesive power and the sealing capacity include acrylic adhesives, e.g. the double coated adhesives no. 9877, no. 1522 or no. 1510 obtainable from 3M Corporate Headquarters, 3M Center, St. Paul, MN 55144-1000, USA, or the transfer adhesive no. 467 or 9471 from the same manufacturer. These examples are not intended to limit the invention to the use of these adhesives but are merely intended to illustrate the diversity and multiplicity of suitable adhesives. Moreover, the suitable adhesives can be provided, not only on the frame, but also on body faceplate. In an alternative embodiment surplus or substitute adhesive may by applied on the body faceplate using e.g. an adhesive applicator designed for this specific purpose, to ensure that a sufficient amount of adhesive always is present.

In a preferred embodiment the frame comprises a collar part, which encircles the magnetic ring, which collar part merges into a radially extending flange part having a lower side provided with the adhesive. This frame structure provides a suitable and effective adherend for keeping the coupling device intimately attached to the body faceplate. The collar part protects the magnetic ring from excessive contact with moisture from the exterior, e.g. when the user swims or showers, thereby prolonging lifetime of the coupling device.

If the flange part extends radially beyond the radial thickness of the collar part towards a central axis of the magnetic ring a shoulder for the magnetic ring is provided. The lower side of the shoulder facing the body faceplate may advantageously be coated with the adhesive so that the magnetic ring can be adhered to the body faceplate in a simple manner. In addition to securing the magnetic ring alongside the internal diameter of the collar part, the magnetic ring can be further secured to the shoulder. Various securing means can be used, all of which must be sufficiently strong to prevent disengagement of the magnetic ring when subjected to axial pulling power during use.

The simplest way of securing the magnetic ring to the collar part and the shoulder is by means of an adhesive. However, within the scope of the present invention the magnetic ring can alternatively be restrained in frictional firm engagement using appropriate dimensioning of the collar part and the magnetic ring. Yet an alternative means is to provide the external circumference of the magnetic ring with a plurality of small projections or a pattern, which infiltrates the wall of the collar part to provide physical anchorage of the magnetic ring to the collar part wall. Combinations and variations of these means are also possible.

By providing the flange part with at least one radially protruding grasping flap the pulling off of the coupling device from a body faceplate is facilitated.

In order to prevent the adhesive from unintentionally adhering to other surfaces than intended during e.g. storage an adhesive on the frame may advantageously be protected by means of a detachable cover sheet.

The coupling device may also comprise a magnetic or magnetic attractable closure cap for temporary closing and coverage of the diameter or aperture of the magnetic ring. In a very simple embodiment the closure cap is a magnetic attractable metal plate having the same outline as the external circumference of the magnetic ring.

The present invention also relates to a drainage assembly for collecting body waste excreted from a body drainage exit, said drainage assembly comprises a body faceplate of the kind having an adhesive body facing side and a body waste receptacle facing side provided with a first magnetic coupling means being the coupling device described above. The drainage assembly further include a body waste receptacle of the kind having a body waste receiving aperture provided with a second coupling means for mating with the first magnetic coupling means on the body faceplate, to provide a leak proof fluid connection between the first coupling means and the second coupling means. The second coupling means is a mate ring of a material allowing the mate ring to magnetically unite with the magnetic ring, and wherein the mate ring is arranged at a body waste receiving aperture of the body waste receptacle and has an outline and configuration adapted for the leak proof and detachably mating with the magnetic ring of the coupling device.

The magnetic ring and the mating ring is axially attracted to each other and held strong together by the magnetic force and provides an body drainage exit channel between the body drainage exit and the body waste receptacle, i.e. a complete closed fluid channel is established. The material of which said two rings are manufactured is chosen to provide an attractive magnetic force of a size that guarantees uncoupling when not intended and allows for uncoupling when intended. Uncoupling can e.g. take place by axially and/or radially displacement of the mate ring in relation to the magnetic ring, or by application of a pull force exceeding the magnetic force.

The drainage assembly may further comprise a mounting part for attaching and fastening the mate ring at the body waste receiving aperture of the body waste receptacle. The mounting part has a tubular part engaging the internal circumference of the mate ring, and a flange part extending radially from the tubular part, said flange part is permanently attached to the body waste receptacle. The mate ring is fitted closely around the tubular part and the flange part of the mounting part has a radial extent, which provide for a permanent, adequate and reliable securing of the mounting part to the body waste receptacle wall. By attaching the flange part to the inner body waste receptacle wall the flange will not separate from the body waste receptacle when the body waste receptacle is subjected to axial uncoupling from the coupling device. It is preferred that the tubular part protrude from the body waste receiving aperture of the body waste receptacle to guide mounting of the mate ring concentric around the tubular part and in use guide the magnetic ring towards the mate ring.

In the preferred embodiment the tubular part has an axial extension, which extends out through the mate ring, and extends inside the magnetic ring when the mate ring and the magnetic ring is magnetically joined to each other. The extended tubular part may in one embodiment advantageously cover the internal diameter of the magnetic ring. As a result direct contact between the magnetic ring and the excreted body waste can be kept as low as possible. Thus, in such an embodiment only a very limited surface area of the magnetic ring will be exposed to the body waste, if any at all, and susceptibility to chemical attacks on the magnetic ring and soiling of the coupling device with body waste can be kept low or even avoided. Consequently, if the magnetic ring and the coupling device is void of body waste, the user is not unpleasantly affected by the sight and touch of the coupling device, and therefore not biased against reuse of said coupling device several times.

If the internal diameter of the magnetic ring and the mating ring is substantially the same a smooth channel between the body drainage exit and body waste receptacle is created. The channel does not disclose any material deposition surfaces for body waste.

The drainage assembly is particular suited for use together with a drainage exit being an artificial stoma selected from ileostomy, colostomy or urostomy.

The present invention also relates to a method of using the drainage assembly described above for collecting bodily waste excreted from a body drainage exit.

The method advantageously comprises the steps of
a) providing a body faceplate having an aperture of a size suitable for surrounding a body drainage exit,
b) securing a coupling device as described above on the body faceplate encircling the aperture of the body face plate,
c) mounting the body faceplate with the coupling device around the body drainage exit on a body surface,
d) detachably mounting the body waste receptacle on the magnetic ring by mating the mate ring and the magnetic ring, and
e) substituting or removing the body waste receptacle according to need.

The user has freedom to make the aperture in the body faceplate according to individual need and anatomical conditions. The subsequent steps of the method are very easy to perform. If the selected body faceplate is of the kind which has a premade punched-out aperture, the aperture of the suitable size, i.e. the size which fit around the body drainage exit, must of course include this premade aperture, but enlargement can be made eccentric to the premade aperture if desired and preferred. If a body faceplate without premade punched-out aperture is selected, the aperture of the suitable size can be made entirely as desired. The user is the one single person knowing his/hers exact need the best and it is therefore a great advantage that he/she has the possibility of designing his/hers own optimum body faceplate, which allows for uncoupling of the body waste receptacle after application.

An incontestability and long-awaited advantage is that because the magnetic contact between the rings will release an audible click noise the method include an inherent, expedient alert to the user that correct coupling has been achieved. Moreover, when the body waste receptacle gets close to the magnetic ring, the magnetic force will attack the mate ring and inherently guide the body waste receptacle in the correct coupling direction.

The method further comprises the steps of
f) disengaging the body faceplate from the body surface in order to substitute the body faceplate,
g) detaching the coupling device from the faceplate, and
h) reusing the coupling device in repeated steps a) - e).

These further steps offer an easy, convenient, and inexpensive method of making use of a magnetic coupling principle, which magnetic principle has hitherto not turned out to find favour among body waste receptacle users.

The method is particular applicable when the body drainage exit is an artificial stoma and the body waste receptacle is an ostomy pouch.

The invention will be explained in greater detail below, describing only exemplary embodiments with reference to the drawing, in which
fig. 1 shows an exploded view, seen in perspective, of the coupling device according to the present invention,
fig. 2 shows the same in assembled state, oblique from a ostomy pouch facing side, where the cover sheet is partly detached from the grasping flap,
fig. 3 shows the same in assembled state oblique from a body faceplate facing side, with the adhesive on the grasping flap exposed,
fig. 4 shows a cross-section taken along the line IV-IV in fig. 2,
fig. 4a shows a modification of the embodiment shown in fig. 4,
fig. 5 shows an exploded view, seen in perspective, of the coupling part, the mate ring and opposing ostomy pouch walls,
fig. 6 shows the same in assembled state, ready for use as an ostomy pouch,
fig. 7 shows a fraction of a cross-section taken along the line VII-VII in fig. 4,
fig. 7a shows, in an enlarged scale, a fragment of fig. 7 illustrating the placement of the mate ring below a lateral protrusion of the extended tubular part of the mounting part,
fig. 8 shows, seen in section, the drainage assembly mounted in relation to a stoma, which protrudes from a skin surface, and
fig. 8a shows in an enlarged scale, a fragment of fig. 8 illustrating the coupling of the mate ring and the magnetic ring.

In the following the invention is described by way of example on the assumption that the body drainage exit is an artificial, abdominal stoma and the body waste receptacle is an ostomy pouch.

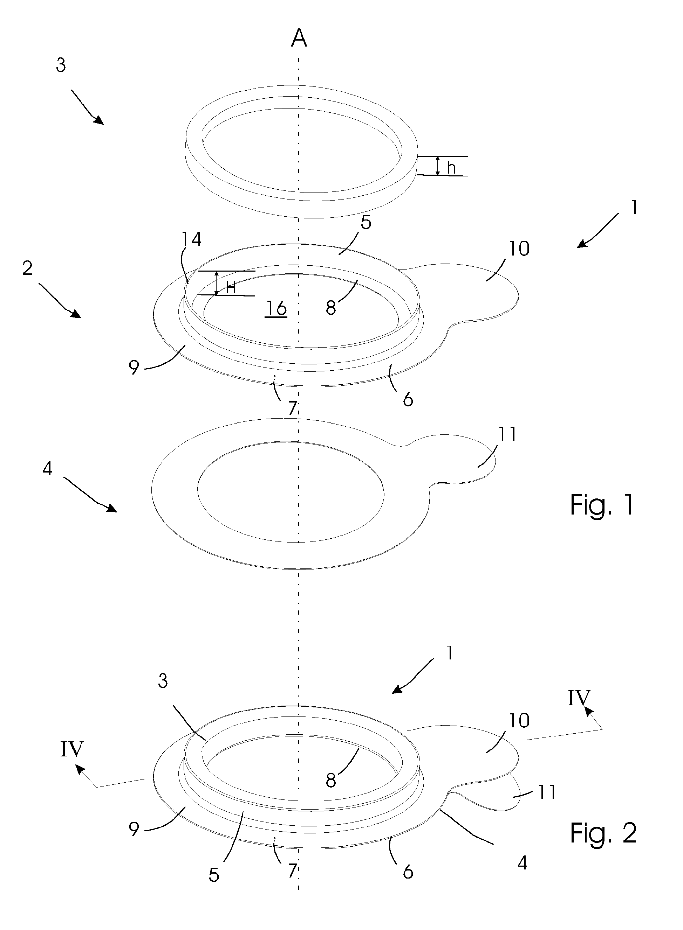

Fig. 1 is an exploded, perspective view of a coupling device 1 according to the present invention. The coupling device 1 is assembled of three parts, a frame 2, a magnetic ring 3 and a cover sheet 4.

The frame 2 has a collar part 5, for encircling the magnetic ring 3 when mounted in the frame 2. The collar part 5 merges into a radially extending flange part 6 having a lower side provided with an adhesive 7. The flange part 6 extends radially in the direction towards a central axis A to provide a shoulder 8 for the magnetic ring 3 in its inserted state in the frame 2. The shoulder 8 prevents a.o. direct contact between the magnetic ring and the skin surface. The part 9 of the flange part 6 which extends in the direction away from the central axis A is provided with a grasping flap 10 protruding radially from the periphery of the part 9.

In the simplest embodiment the magnetic ring 3 is a solid, ferromagnetic ring 3 having a thickness h that corresponds substantially to the axial internal heigth H of the collar part, i.e. the internal distance from the shoulder to the free edge of the collar part. However, these lengths can be longer or shorter if more appropriate for a particular design and use.

The adhesive 7 is preferably selected among adhesives, which can be attached and detached numerous times for reuse of the coupling devise 1. Suitable adhesives are listed above. During storage and at least before the first use the adhesive 7 is protected by the detachable cover sheet 4. Surplus or substitute adhesive can be applied on either the lower side of the frame or on the body faceplate if required.

The frame 2 is shown as a circular frame, however any outline can be chosen as long as the magnetic ring 3 is provided with a corresponding outline that allows this magnetic ring 3 to fit tight inside the frame 2.

In fig. 2 the assembled coupling device is shown in perspective, oblique from a pouch facing side. The magnetic ring 3 is inserted in the collar part and secured, e.g. adhered, to the shoulder 8 and the internal diameter of the collar part to provide a solid, leak-proof anchoring of the magnetic ring 3 inside the frame 2. A cover flap 11 of the cover sheet 4 is folded back to illustrate the detachability of the cover sheet 4.

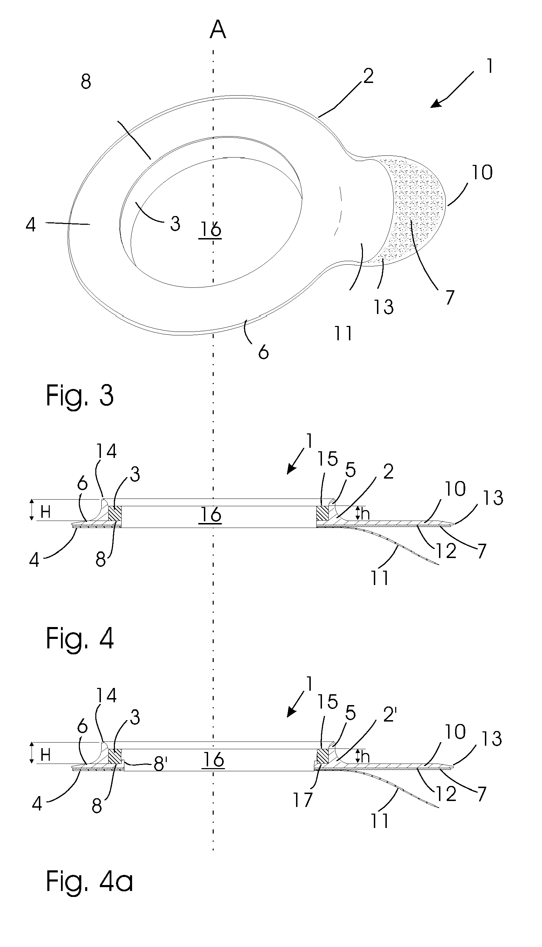

The back folding of the cover flap 11 is also illustrated in fig. 3, in which the coupling device 1 is seen in perspective, oblique from a body faceplate side 12 of the frame 2. As indicated with a shaded signature at the exposed part of the grasping flap 10, the adhesive 7 is distributed over the body faceplate side 12 of the frame 2 so that a secure and firm detachable attachment to a body faceplate can be achieved. Although in fig. 3 a tiny rim 13 is left free of adhesive 7, this is optional and within the scope of the present invention total coverage of the flange with adhesive 7 is preferred. However, at the grasping flap 10 it may be preferred to leave a small rim portion 13 or even the entire grasping flap 10 free of adhesive 7 to facilitate grasping of the grasping flap 10 when the coupling device 1 is to be detached for reuse.

Fig. 4 shows a sectional view of the coupling device 1 taken along the line IV-IV in fig. 2. As seen better in this figure the height H of the collar part is greater than the thickness h of the magnetic ring, so that the free end 14 of the collar part 5 protrude a distance beyond the free edge 15 of the magnetic ring 3. Fig. 4 further illustrates that the adhesive 7 covers substantially the entire body faceplate side 12 of the frame 2 and that the magnetic ring 3 is fitted tightly inside the collar part 5 and rests on the shoulder 8.

Fig. 4a includes a modification of the design of the frame 2 shown in fig. 4. The modified frame 2' has a shoulder 8 which is provided with a jut 8' extending substantially parallel to the axis A, to provide a cavity 17 in which the magnetic ring 3 can be effectively mounted.

The free end 14 of the collar part 5 can also be provided with a straight upper edge or be provided with a small recess to engage with a corresponding protruding part on associated coupling means on the drainage pouch, so that an additional coupling aid is provided.

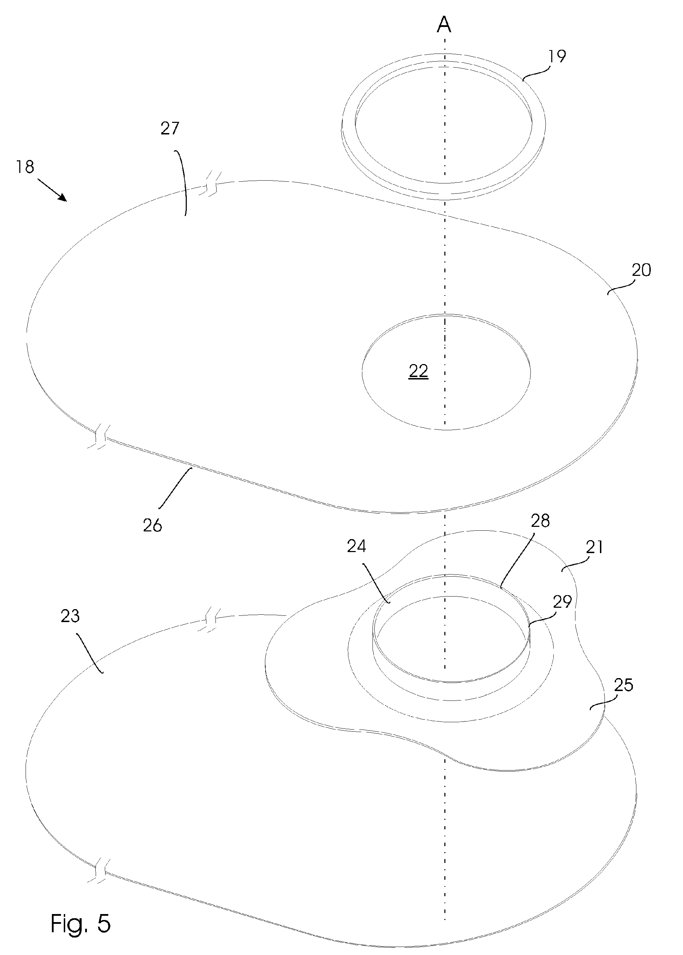

Fig. 5 shows a perspective, exploded view of a body waste receptacle 18 in the form of an ostomy pouch 18. The ostomy pouch 18 is constructed of a second coupling means, which is a mate ring 19, and an inner pouch wall 20, a mounting part 21 for firmly attaching the mate ring 19 at a body waste receiving aperture 22 of the inner pouch wall 20, and an outer pouch wall 23. The mounting part 21 has a tubular part 24 and a flange part 25 extending radially from this tubular part 24.

The assembled ostomy pouch 18 is manufactured by passing the tubular part 24 of the mounting part 21 through the body waste receiving aperture 22 of the inner pouch wall 20, so that the tubular part 24 protrudes from the aperture 22. The flange part 25 is then attached to the inner side 26 of the inner pouch wall 20 by means of e.g. an adhesive or by welding, so that the anchoring of the mounting part is both solid and leak-proofed. Subsequently, the mate ring 19 is arranged around the tubular part 24 with the internal diameter of the mate ring 19 in engagement with the external diameter of the tubular part 19. The tubular part has an axial extension 28 with a lateral protrusion 29. The lateral protrusion 29 extends to overlap the mate ring 19 when the mate ring is mounted around the tubular part 24, and the lateral protrusion 29 retains the mate ring 19 on the tubular part when the ostomy pouch 18 is uncoupled from its magnetic engagement with the magnetic ring 3. Further, the mate ring 19 can be firmly secured to the outer side 27 of the inner pouch wall 20 by gluing. Finally, the inner pouch wall 20 and the outer pouch wall 23 is joined, e.g. by welding, along their circumferences to provide a closed ostomy pouch 18 provided with a second coupling means 19, which is magnetical attractable.

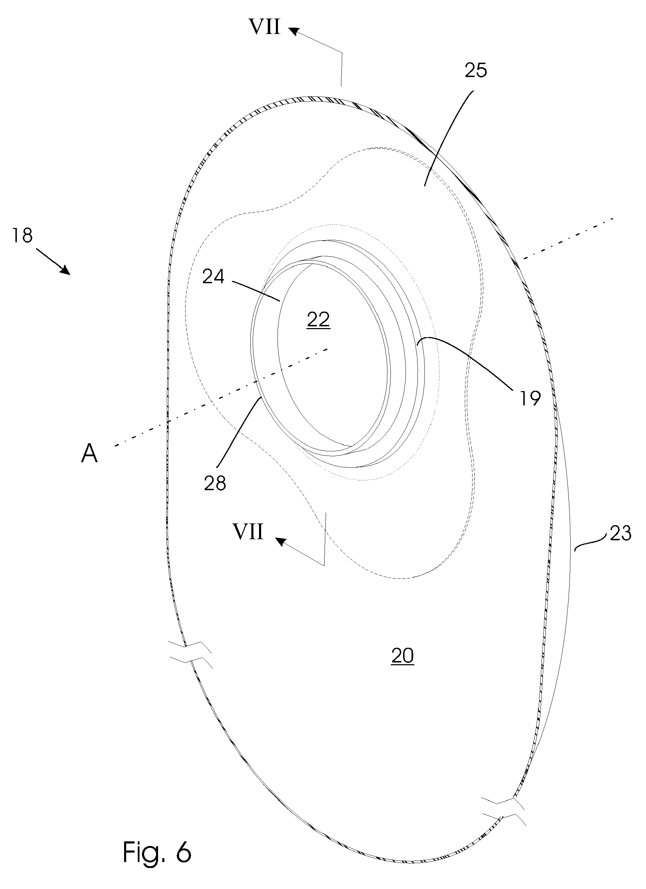

The assembled ostomy pouch 18 is seen better in fig. 6, in which the placing of the mounting part 21 inside the ostomy pouch 18 is indicated in dotted line. The flange part 25 of the mounting part 21 is in the figures shown to be of a triangular outline, however within the scope of the present invention the outline can be of any appropriate shape, such as circular, quadratic or other polygonal shape. Also, the radial extent of the flange part 25 may be chosen depending on e.g. the type of drainage pouch, the pouch wall material, the size of the pouch, etc. The ratio of dimensions indicated in the figures are not to be construed as limiting the present invention.

The flange part 25 advantageously also serves as a retainer plate. Said flange part 25 can alternatively be introduced through the aperture 22 after the pouch walls 20,23 are joined together.

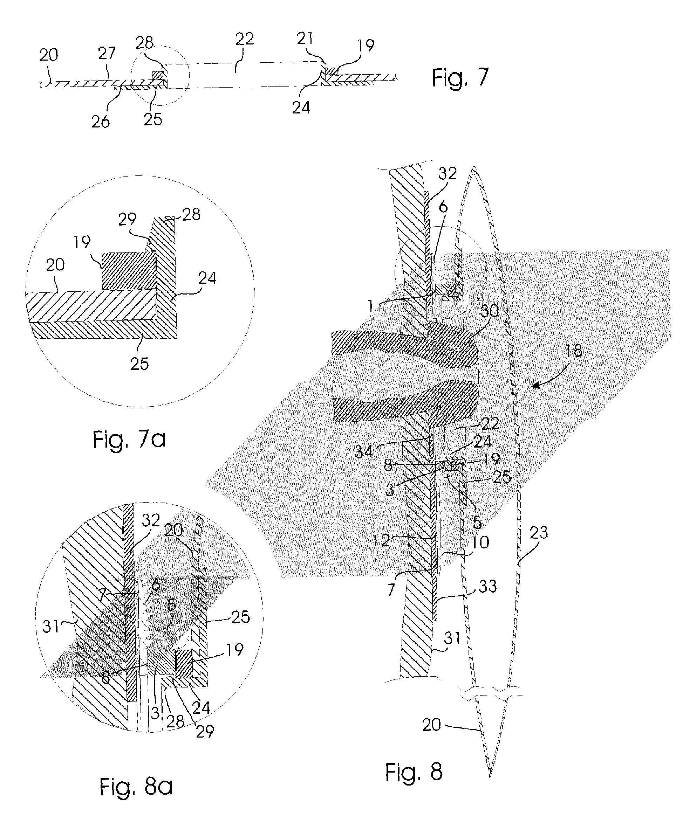

The extension 28 and the lateral protrusion 29 of the tubular part is seen better in fig. 7, which is a fractional, sectional view taken along line VII-VII in fig. 6.

As seen more clearly in the enlarged fractional, sectional view in fig. 7a the mate ring 19 is kept below the lateral protrusion 29. The extension 28 tapers in the axial direction to enable easy, and firm snap-fitting of the mate ring 19 around the tubular part 24 and down below the extension 28.

Fig. 8 illustrates the drainage assembly mounted on a stoma 30, which protrudes from a body surface 31.

A body faceplate 32 is provided with a coupling device 1 on the side 33 of the body faceplate 32 and encircles the aperture 34 of the body faceplate 32. The joined body faceplate 32 and coupling device 1 is adhered to the body surface 31 so that apertures 16,22 surround the stoma 30. When the ostomy pouch 18 is approached to the stoma, which is surrounded with the coupling device furnished body faceplate, the mate ring 19 is magnetically directed towards the magnetic ring 3. Once the rings 3,19 meet they are forceably held together by the magnetic attractive force of the magnetic ring. The coupling together of the two rings 3,19 releases an audible click noise alerting the user that a perfect coupling has been obtained.

As can be seen more clearly in the enlarged fractional, sectional view of fig 8a, the heights of the collar part 5, the tubular part 24, and the thickness of the magnetic ring 3 and the mate ring 19 can be adjusted so that e.g. the height of the tubular part 24 is long enough to provide for the axial extension 28 to meet the shoulder 8 so that contact between the reusable, magnetic ring 3 and any chemical aggressive body waste, such as faeces or urine, is kept as low as possible. The height H of the collar part 5 is preferably selected to be substantially equal to or smaller than the sum of the thickness of the magnetic ring 3 and the mate ring 19.

Within the scope of the present invention the design of the various parts can be modified. As examples of modifications, can be mentioned that the size of the ostomy pouch can be made both smaller and larger. The pouches can be of the kind, which can be emptied. The apertures 16,22,34 may be made smaller to fit tighter around the stoma 30 and the coupling device 1 may be configured without grasping flap.

Both the magnetic ring and the mate ring can be of magnetic material, however since a magnetic material is expensive the advantages of the invention is a.o. seen in the reusability of the magnetic coupling device together with a disposable ostomy pouch provided with a cheap magnetic attractable metal ring.

The drainage assembly can be used for other kinds of drainage exits than an artificial stoma. The drainage assembly could e.g. be used in abdominal dropsy or be arranged in relation to continuously collecting purulent matter from an abscess. This list is not exhaustive.

## Claims

1. A coupling device (1) for providing an adhesive body faceplate (32) with means for connecting the body faceplate (32) to a body waste receptacle (18), the body waste plate (32) has an aperture (24) adapted to register with a body drainage exit (30), **characterised in that** the coupling device (1) comprises
- a frame (2) having a central aperture (16) adapted to register with the body drainage exit (30),
- a magnetic ring (3) mounted in the central aperture (16) of the frame (2) at the internal circumference of the central aperture (16), and
- that any of the frame (2) or the body faceplate (32) are provided with means (7) for detachable attachment of the coupling device (1) to the body faceplate (32).

2. A coupling device (1) according to claim 1, **characterised in that** the means (7) for detachable attachment of the coupling device (1) to the body faceplate (32) is an adhesive (7).

3. A coupling device (1) according to claims 1 or 2, **characterised in that** the frame (2) comprises a collar part (5), which encircles the magnetic ring (3), which collar part (5) merges into a radially extending flange part (6) having a lower side provided with the adhesive (7).

4. A coupling device (1) according to claim 3, **characterised in that** the flange part (6) extends radially beyond the radial thickness of the collar part (5) towards a central axis of the magnetic ring.

5. A coupling device (1) according to claims 3 or 4, **characterised in that** the flange part (6) has at least one radially protruding grasping flap (10).

6. A coupling device (1) according to any of the preceding claims 2 - 5, **characterised in that** the adhesive (7) is protected by a detachable cover sheet (4).

7. A drainage assembly for collecting bodily waste excreted from a body drainage exit (30), said drainage assembly comprises
- a body face plate (32) of the kind having an adhesive body facing side and a body waste receptacle facing side provided with a first magnetic coupling means (1), and
- a body waste receptacle (18) of the kind having a body waste receiving aperture (22) provided with a second coupling means for mating with the first magnetic coupling means on the body face plate (32), to provide a leak proof fluid connection between the first coupling means and the second coupling means,
**characterised in that**
- the first magnetic coupling means is the coupling device (1) according to any of the claims 1 - 6, and
- the second coupling means is a mate ring (19) of a material allowing the mate ring to magnetically unite with the magnetic ring (3), and
- the mate ring (19) is arranged at a body waste receiving aperture (22) of the body waste receptacle (18) and has an outline and configuration adapted for the leak proof and detachably mating with the magnetic ring (3) of the coupling device (1).

8. A drainage assembly according to claim 7, **characterised in that** the drainage assembly further comprises a mounting part (21) for attaching the mate ring (3) at the body waste receiving aperture (22) of the body waste receptacle (18), the mounting part (21) has a tubular part (24) engaging the internal circumference of the mate ring (3), and a flange part (25) extending radially from the tubular part (24), said flange part (25) is permanently attached to the body waste receptacle (18).

9. A drainage assembly according to claim 8, **characterised in that** at least the flange part (25) is attached to the body waste receptacle (18) inside the body waste receptacle (18) and the tubular part (24) protrudes from the body waste receiving aperture (22) of the body waste receptacle (18) .

10. A drainage assembly according to claims 8 or 9, **characterised in that** the tubular part (24) has an axial extension (28), which extends out through the mate ring (19), and extends inside the magnetic ring (3) when the mate ring (19) and the magnetic ring (3) is magnetically joined to each other.

11. A drainage assembly according to any of the preceding claims 8 - 10, **characterised in that** the drainage exit is an artificial stoma (30) selected from ileostomy, colostomy or urostomy.

12. A method of using the drainage assembly according to any of the preceding claims 8 - 10 for collecting bodily waste excreted from a body drainage exit (30).

13. A method according to claim 13, **characterised in that** the method comprises the steps of
a) providing a body face plate (32) having an aperture (34) of a size suitable for surrounding a body drainage exit (30),
b) securing a coupling device (1) according to any of the claims 1 - 6 on the body face plate (33) encircling the aperture (34) of the body face plate (32),
c) mounting the body face plate (32) with the coupling device (1) around the body drainage exit (30) on a body surface (31),
d) detachably mounting the body waste receptacle (18) on the magnetic ring (3) by magnetically mating the mate ring (19) and the magnetic ring (3), and
e) substituting or removing the body waste receptacle (18) according to need.

14. A method according to claims 13 - 14, **characterised in that** the method further comprises the steps of
i) disengaging the body faceplate (32) from the body surface (31) in order to substitute the body face plate,
j) detaching the coupling device (1) from the body faceplate (32), and
k) reusing the coupling device (1) in repeated steps a) - e) .

15. A method according to any of the claims 12, 13, or 14, **characterised in that** the body drainage exit is an artificial stoma (30) and the body waste receptacle is an ostomy pouch (18).
